(19) Europäisches Patentamt / European Patent Office / Office européen des brevets

(11) EP 2 561 856 B1

(12) EUROPEAN PATENT SPECIFICATION

(45) Date of publication and mention of the grant of the patent:
**25.10.2017 Bulletin 2017/43**

(21) Application number: **11181559.3**

(22) Date of filing: **16.09.2011**

(51) Int Cl.:
*A61K 8/46* (2006.01) *A61K 8/81* (2006.01)
*A61Q 9/04* (2006.01)

# (54) Method and kit for depilation

Verfahren und Kit zur Depilation

Procédé et kit d'épilation

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.08.2011 US 526699 P**

(43) Date of publication of application:
**27.02.2013 Bulletin 2013/09**

(73) Proprietor: **The Procter & Gamble Company**
**Cincinnati, OH 45202 (US)**

(72) Inventors:
- **Smith, Charles, Robert**
  **Swindon, Wiltshire SN1 5LA (GB)**
- **Hewlins, Stuart, Andrew**
  **Woking, Surrey GU24 9LW (GB)**
- **Goffe, Michael, John**
  **Egham, Surrey TW20 9LF (GB)**

(74) Representative: **Kohol, Sonia**
**Technical Centres Limited**
**Procter & Gamble Patent Department**
**Rusham Park**
**Whitehall Lane**
**Egham, Surrey TW20 9NW (GB)**

(56) References cited:
**EP-A1- 2 368 541** **EP-A1- 2 368 542**
**WO-A1-2004/096164** **WO-A1-2009/090362**

- **DATABASE GNPD [Online] Mintel; May 2010 (2010-05), "Soothing Hair Removal Face Cream", XP002665851, Database accession no. 1327960**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

FIELD OF THE INVENTION

The present invention relates to a method and kit for depilation.

BACKGROUND OF THE INVENTION

Depilatory compositions are cosmetic hair removal formulations. They comprise keratin reducing agents, which attack the disulphide bonds in hair to weaken it, such that subsequent gentle scraping and/or wiping completes severance of the hair from the skin and effects hair removal. Commercially, the most common keratin reducing agents are thioglycolates, which are typically formulated at high pH. An unwanted side effect of chemical depilation is that the depilatory composition comes into contact with and must have a relatively long residence time on skin to achieve effective hair removal and this long residence time combined with the alkaline conditions needed for effective hair removal may give rise to skin irritation.

The above problem has been recognized in the art. Reference is made to US 4,401,663 and US 4,424,205 the disclosures of which are similar to one another. These documents teach to use certain compounds as topical analgesics and an example given of a situation in which it is suggested to use such materials is to reduce depilatory irritation. The carrier formulas disclosed to be suitable for formulation with the analgesics include lotions and creams.

Reference is also made to US 2004/0219118, which discloses treatment with a "lipophilic" material before application of a thioglycolate-based reactive depilatory compostion. Lipophilic materials exemplified in this patent application are oils, such as mineral oil. As shown hereinbelow, the present applicants have tested a range of lipophilic materials to determine their ability to prevent thioglycolate penetration and, thereby, their ability to reduce or prevent skin irritation Applicants have surprisingly found that oils, such as mineral oil, have no or a low ability to prevent thioglycolate penetration to the skin. There thus exists a need to develop a pre-treatment composition which better reduces skin irritation.

SUMMARY OF THE INVENTION

According to a first aspect of the invention, a depilatory kit is provided, comprising:

(a) a protective composition, the protective composition comprising from 1% to 50% copolymer by weight of the protective composition, the copolymer comprising at least 50% by weight of the copolymer of monomers which have hydrophobic side-groups, each hydrophobic side-group comprising from 8 to 40 carbon atoms, as defined in claim 1, and

(b) a depilatory composition comprising an effective amount of a keratin reducing agent, comprising a thioglycolate acid salt.

According to a second aspect of the invention, a method of removing hair from skin is provided, comprising the steps of:

(a) applying the protective composition defined in the first aspect of the invention to an area of skin on which unwanted hair is growing;

(b) applying a depilatory composition to the area of skin to which the protective composition has been applied, the depilatory composition comprising a keratin reducing agent.

BRIEF DESCRIPTION OF THE DRAWINGS

Fig.1. is a schematic view of a Franz Cell apparatus.

DETAILED DESCRIPTION OF THE INVENTION

The protective composition used in the method and comprised within the kit according to the invention comprises from 1% to 50% copolymer by weight of the protective composition, the copolymer comprising at least 50% by weight of the copolymer of monomers which have hydrophobic side-groups comprising from 8 to 40 carbon atoms. As demonstrated by the Franz Cell test data, below, the applicants have established that such protective compositions are significantly better at preventing the penetration of keratin reducing agent, such as thioglycolate, to the skin, than oil on its own and therefore result in reduced skin irritation. Applicants have established that the use of the present protective composition still permits hair weakening and destruction by the keratin reducing agent, so that reduced skin irritation can be achieved while removing hair. A reduction of thioglycolic acid penetration of 45% or more according to the Franz Cell test method may be shown to correlate to a significant and user-noticeable reduction in irritation.

The protective composition used in the method and comprised within the kit according to the invention comprises from 1% to 50%, preferably from 2% to 30%, more preferably from 3% to 20% of the copolymer by weight of the protective composition.

According to the invention, the copolymer comprises at least 50%, preferably from 50% to 95%, more preferably from 60% to 90% by weight of the copolymer of monomers with hydrophobic side-groups. The side-groups comprise from 8 to 40, preferably from 8 to 30 carbon atoms. Advantageously, the hydrophobic side-groups are straight chain alkyl groups. These side-groups render the copolymer hydrophobic in nature.

Advantageously, the carbon-carbon bonds within the hydrophobic side-groups have a degree of unsaturation of less than 50%, preferably less than 25% and are more preferably fully saturated. The percentage degree of unsaturation (%DU) may be calculated using the following formula:

$$\%DU = \left(1 + \sum \frac{(N°(C-C)Double\ Bonds) + (2 \times N°(C-C)Triple\ Bonds)}{N°\,Carbons}\right) \times 100$$

wherein:

- the term "N°(C - C) Double Bonds" refers to the number of double bonds with the hydrophobic side-chain
- the term "N°(C - C) Triple Bonds" refers to the number of triple bonds with the hydrophobic side-chain
- the term "N° Carbons" refers to the number of carbon atoms with the hydrophobic side-chain

According to one embodiment according to the invention, the copolymer comprised within the protective composition comprises monomers having formula (monomer A):

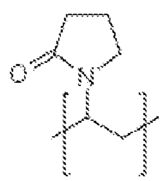

and monomers having formula (monomer B):

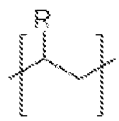

where R is a hydrophobic side-group comprising a straight alkyl chain having from 12 to 30 carbon atoms and wherein the copolymer comprises from 50% to 99%, more preferably 50% to 90% by weight of monomer B with the remainder being monomer A.

According to another embodiment according to the invention, the copolymer comprised within the protective composition comprises monomers having formula (monomer D):

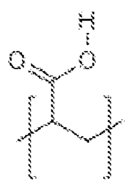

and monomers having formula (monomer E):

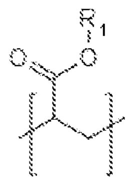

where $R_1$ is a hydrophobic side-group comprising a straight alkyl chain having from 10 to 30 carbon atoms; and monomers having formula (monomer F):

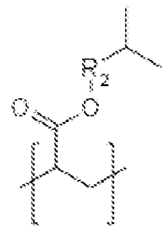

where $R_2$ is a hydrophobic side-group comprising a straight alkyl chain having from 4 to 26 carbon atoms, wherein the copolymer comprises from 50% to 99%, more preferably 50% to 90% by weight of the combination of monomers E and F, with the remainder being monomer D.

The protective composition may comprise a single phase or more than one phase.

If the protective composition comprises a single phase, then, given the hydrophobic nature of the copolymer, the protective composition will essentially a single hydrophobic phase (although it may comprise a few cosmetic adjuncts dispersed within it, which are not hydrophobic). In this case, the single phase protective composition may additionally comprise other hydrophobic materials, such as oils, waxes and triglycerides (fats).

If the protective composition comprises more than one phase, then it may be in the form of an emulsion, which may have a hydrophilic continuous phase and a hydrophobic dispersed phase or a hydrophobic continuous phase and a hydrophilic dispersed phase. The copolymer is comprised within the hydrophobic phase, whether that phase is the dispersed or the continuous phase. As in the case in which the protective composition comprises a single phase, the hydrophobic phase may comprise other materials mixed with the copolymer, such as oils, waxes, triglycerides (fats), oil soluble skin active agents and mixtures thereof.

Oils which may be mixed with the copolymer to form the hydrophobic phase, whether part of a single or multi-phase composition, include natural oil, synthetic oil, silicone oil and mixtures thereof.

Waxes which may be mixed with the copolymer to form the hydrophobic phase, whether part of a single or multi-phase composition, include natural wax, synthetic wax, silicone wax and mixtures thereof.

Triglycerides which may be mixed with the copolymer to form the hydrophobic phase, whether part of a single or multi-phase composition, have the following formula:

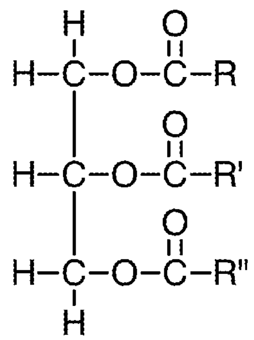

wherein R, R' and R" may be the same as or different from one or both of the others, wherein each of R, R' and R" is a fatty acid.

Oil soluble skin active agents which may be mixed with the copolymer to form the hydrophobic phase, whether part of a single or multi-phase composition, oil soluble vitamins, such as vitamin E derivatives, including vitamin E acetate and tocopherol nicotinate; oil-soluble vitamin A derivatives, such as retinyl palmitate; lanolin; ceramides; sterols and sterol esters; salicylic acid; camphor; eucalyptol; essential oils and mixtures thereof.

The protective composition used in the method and comprised within the kit according to the invention may include small quantities of further ingredients such as, but not limited to metal oxides, organic and inorganic dyes, lakes, micas, flavourings, perfumes and mixtures thereof.

If the protective composition used in the method and comprised within the kit according to the invention comprises an emulsion, then it advantageously comprises from 4.9% to 89.9%, preferably from 10% to 75%, more preferably from 25% to 65% hydrophilic phase by weight of the protective composition.

The hydrophilic phase typically comprises water, but need not. In addition to or as an alternative to water, the hydrophilic dispersed phase may comprise hydrophilic materials, such as polyhydric alcohols, ethoxylated and propoxylated polyols, polysaccharides, and mixtures thereof. Suitable polyhydric alcohols (polyols) include, but are not limited to, butylene glycol, hexylene glycol, ethoxydiglycol, dipropylene glycol, phenyl ethyl alcohol, glycerin, 1,3-butanediol, 1,2-propanediol, isoprene glycol, sorbitol, polyethylene glycol, polypropylene glycol and mixtures thereof. Preferred polyols include glycerin, propylene glycol, panthenol and mixtures thereof.

Additionally, the hydrophilic phase may comprise other polar solvents, such as alcohols, ketones and mixtures thereof. Examples of suitable polar solvents include phenyl ethyl alcohol, ethanol, isopropyl alcohol and mixtures thereof.

The hydrophilic phase may additionally comprise hydrophilic skin active agents such as, but not limited to, vitamins, including hydrophilic ascorbic acid compounds and vitamin B3 compounds; azelaic acid; gallic acid and its derivatives; N-acetyl glucosamine; panthenol and mixtures thereof.

If the protective composition used in the method and comprised within the kit according to the invention is an emulsion then it will comprise an appropriate emulsifier selected by the skilled person, according to the type of emulsion in question. The protective composition will typically comprise from 0.1% to 10%, and preferably from 0.1% to 5% emulsifier by weight of the protective composition. The emulsifier may be nonionic, anionic or cationic. Suitable emulsifiers are disclosed in McCutcheon's Detergents and Emulsifiers, North American Edition, pages 317-324 (1986).

Any depilatory composition comprising a suitable keratin reducing agent may be used in the present method and included in the present kit. Non-limiting examples of suitable keratin reducing agents include: sulphide salts such as $Li_2S$, $Na_2S$, $K_2S$, MgS, CaS, SrS or BaS, hydrogen sulphide salts such as NaSH or KSH; thioglycol; thioglycerol; thioglycolamide; thioglycolhydrazide; thioglycolic acid; thioglycolate salts (such as potassium thioglycolate, calcium thioglycolate, ammonium thioglycolate, diammonium dithioglycolate, glyceryl monothioglycolate, or monoethanolamine thioglycolate); thiosalicylic acid; thiomalic acid; ammonium thiolactate; monoethanolamine thiolactate; dithioerythritol; 2-mercaptopropionic acid; 1,3-dithiopropanol; glutathione; dithiothreitol; cysteine; homocysteine; N-acetyl-L-cysteine and cysteamine. Advantageously, the keratin reducing agent is comprised within the depilatory composition in an amount from 0.3% to 20%, preferably from 0.8% to 15%, more preferably from 1% to 10% by weight of the depilatory composition.

Advantageously, the depilatory composition may comprise at least one thioglycolate salt or thioglycollic acid acting as a hair removal agent when the depilatory composition is applied to unwanted hair. Preferably, the depilatory composition comprises sodium, potassium, magnesium, calcium, beryllium, strontium, zinc, monoethanolamine, ammonium, tetralkylammonium, imidazolium, pyridinium, phosphonium or glyceryl thioglycolate salts, or mixtures thereof, which may include dianion forms of thioglycolate. More preferably, the depilatory composition comprises at least one of sodium, potassium, magnesium or calcium thioglycolate, or mixtures thereof. Even more preferably the depilatory composition comprises potassium or calcium thioglycolate, or mixtures thereof.

The pH of the depilatory composition may advantageously be in the range of from 6 to 13.8, preferably from greater than 7 to 13, more preferably from 9 to 12.9, even more preferably from 10 to 12.8, even more preferably still from 12 to 12.75 and yet more preferably from 12.3 to 12.6 to improve the efficacy of the active ingredient. The depilatory composition may, in a preferred embodiment, comprise at least one base to control the pH. Preferably, the depilatory composition comprises potassium hydroxide; sodium hydroxide; lithium hydroxide; calcium hydroxide; barium hydroxide; caesium hydroxide; sodium hydroxide; ammonium hydroxide; strontium hydroxide; rubidium hydroxide; magnesium hydroxide; zinc hydroxide; sodium carbonate; pyridine; ammonia; alkanolamides (including monoethanolamine, diethanolamine, triethanolamine), phosphates (including tetrasodium phosphate), arginine or mixtures thereof. More preferably, the depilatory composition comprises at least one buffering base, even more preferably the depilatory composition comprises calcium hydroxide, magnesium hydroxide; barium hydroxide; strontium hydroxide; zinc hydroxide; arginine or mixtures thereof. Still more preferably the depilatory composition comprises calcium hydroxide; magnesium hydroxide, zinc hydroxide, sodium hydroxide, potassium hydroxide or mixtures thereof. Even more preferably still, the depilatory composition comprises calcium hydroxide, sodium hydroxide or mixtures thereof.

In an advantageous embodiment, the base is present at a concentration of from 0.1% to 10.0%, more preferably from 0.5% to 8.0% and even more preferably from 1.0% to 5.0%, by weight of the depilatory composition.

The concentration of water in the depilatory composition is preferably at least 40%, more preferably from 50% to 98%, even more preferably from 60% to 95% and even more preferably still from 70% to 90%, by weight of the depilatory composition.

The depilatory composition may optionally comprise a thickening agent. A representative but not exhaustive list can be found in "The Encyclopaedia of Polymers and Thickeners for Cosmetics" compiled and edited by Robert Y.

Lochhead, PhD and William R. Fron, Department of Polymer Science, University of Southern Mississippi. Exemplary classes of thickening agents include gums, carbomers, polymers and copolymers of acrylic acid, associated thickeners, layered silicates/clays and natural polymers (including polysaccharides). One or more thickening agents may be included in the aqueous depilatory composition. The thickening agent may be present at a level of from about 0.01% to about 20%, preferably from about 0.1% to about 10% by weight of the depilatory composition.

The depilatory composition may also include other skin care ingredients such as conditioning agents selected from the group consisting of humectants, moisturizers, or skin conditioners (including mineral oil; almond oil; chamomile oil; jojoba oil; avocado oil; shea butter, niacinamide and glycerine); skin rejuvenation compositions (for example targeted for fine lines, wrinkles and uneven skin tone, including retinoids), cosmetic compositions; anti-inflammatory agents (including corticosteroids); anti-oxidants (including flavonoids) radical scavengers; sunscreen agents; skin cooling or warming agents and the like. The depilatory composition may comprise one or more skin care ingredients present in an amount of from about 0.001% to about 10%, more preferably from about 0.01% to about 7%, and even more preferably from about 0.025% to about 5%, by weight of the depilatory composition.

An accelerant may be employed in the depilatory composition. This optional component accelerates the rate of depilatory action of the depilatory agent. Suitable accelerants include, but are not limited to, urea; thiourea; dimethyl isosorbide; arginine salts; ethoxydiglycol; propylene glycol and methylpropyldiol. The accelerant may be present in a concentration range of from 0.5% to 10%, more preferably from 2% to 8% and even more preferably from 2% to 5% by weight of the depilatory composition.

The depilatory composition may further comprise components known, conventionally used, or otherwise effective for use in cosmetic compositions, such as dyes; pigments (including ultra marines and talc); anionic, cationic, nonionic and/or amphoteric or zwitterionic surfactants, polymers (including hydrophobically modified polymers); dispersing agents; solvents; lubricants; fragrances; preservatives; chelants, proteins and derivatives thereof, plant materials (e.g. aloe, chamomile and henna extracts); silicones (volatile or non-volatile, modified or non-modified); film-forming agents; film forming promoters and mixtures thereof.

The depilatory composition may be formulated in any common delivery form, such as a cream or lotion. Alternatively, it may be delivered on a substrate, such as a thin film of depilatory composition coated onto the substrate. The substrate may be configured in any suitable form, such as a strip, mask or patch.

In addition to the protective composition and the depilatory composition, the kit according to the second aspect of the invention may comprise one or more of:

(a) A make-up removal composition and/or a make-up removal wipe;

(b) Means for removal of the protective composition and the depilatory composition following use, which means may comprise one or more of a tool, such as a scraper or a spatula; or a wipe;

(c) A post-treatment composition skin care composition to be applied to the area of skin from which hair has been removed. Such a post-treatment skin care composition may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like. The post-treatment skin care composition may be leave-on or a rinse-off composition.

(d) Instructions regarding how to use the various elements of the kit, which instructions may comprise one or more elements of the method as defined herein.

Prior to applying the method or using the kit according to the present invention, a user should advantageously remove all make-up from the skin, to ensure good adherence and effective application of both the protective composition and the depilatory composition.

The method according to the first aspect of the invention comprises the step of applying the above-defined protective composition to an area of skin on which unwanted hair is growing, which may be located on any part of the human body.

Advantageously, the protective composition is not just applied to the area to be depilated, but also to an immediately juxtaposing area thereabout (that is, the protective composition is applied to an area of skin which is greater than just the area which is to be depilated).

Advantageously, the user will apply from 0.3 - 2mg of protective composition per square centimetre of skin, preferably from 0.4 - 1.3mg/cm$^2$, more preferably from 0.4 to 1mg/cm$^2$.

Following application, the protective composition is advantageously massaged into the skin. Preferably, massaging is effected for at least 10 seconds, and, more preferably, massaging is effected as a circular motion. Without wishing to be bound by theory, it is believed that the protective composition may trap hair within it thereby shielding it

from the to-be-applied depilatory composition; massaging may help to release the hairs from the skin and ensure improved access thereto by the depilatory composition.

The method according to the first aspect of the invention comprises the subsequent step of applying the above-defined depilatory composition to an area of skin on which unwanted hair is growing and to which protective composition has already been applied. Advantageously, the user will apply a layer of depilatory composition which is from 0.1mm to 5mm, preferably from 0.3 to 3mm, more preferably from 0.5 to 2mm in thickness.

Subsequently, according to the method of the first aspect of the invention, the depilatory composition is advantageously left in place for at least 1 minute, preferably from 1 to 10 minutes, more preferably from 3 to 10 minutes, depending on the thickness of the hair and the hair removal efficacy of the depilatory composition (which, in turn, is dependent upon the concentration of keratin reducing agent in the depilatory composition).

Subsequently, according to the method of the first aspect of the invention, the protective composition and the depilatory composition are advantageously removed. This may be achieved using one or more of a cotton wool ball, pad or wand, a tissue, a cloth, or a tool, such as a spatula or a scraper. Advantageously, the skin from which hair has been removed is then rinsed with water.

In an advantageous subsequent step, a post-treatment skin care composition may be applied to the area of skin from which hair has been removed. Such a post-treatment skin care composition may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like. The post-treatment skin care composition may be leave-on or a rinse-off composition.

Franz cell method

Principle and Scope:

This method is applicable for using Franz cell apparatus for the *in-vitro* assessment of penetration of thioglycolic acid (TGA) and its salts through a skin mimic after the application of a depilatory composition following pre-treatment with a protective composition.

Penetrated TGA is quantified using Reverse Phase High Performance (or Pressure) Liquid Chromatography (RP-HPLC) with external standard quantitation at 240nm.

Method

Reference is made to Figure 1 and to the reference numerals therein:

1. Prepare the Vitro-Skin (IMS Vitro-Skin®, Catalogue number: P&G1013, made by IMS Inc., Portland, Maine, USA) samples by cutting 8x6.2cm segments and placing them textured side up on the racks into a hydration chamber (manufactured & sold by IMS) containing a 14.7% glycerol solution. The hydration chamber should be sealed and the vitro-skin left to hydrate at room temperature and a humidity of 80.4% ±3.5% for 24 hours.

2. Prepare the receptor solution for the Franz-cell by mixing 1.90ml formic acid (98%wt+ Fluka, by Sigma Aldrich, or equivalent), 30ml acetonitrile (RP-HPLC grade) and 968.1ml water (RP-HPLC grade). Set up the static Franz cell (Permegear or equivalent, 15mm diameter unjacketed cell with a 12ml receptor volume) by clamping it in place over suitable stirrer plates (not shown) and add a small stirrer bar (6) to each cell, fill the receptor cell (2) to the brim with the required amount of receptor solution.

3. Once hydrated, remove a sheet of vitro-skin from the hydration chamber and lay textured side up on a clean flat surface then dose 100μl (~2mg/$cm^2$) of protective composition (not shown) onto the vitro-skin and spread evenly over the surface by rubbing for 30 seconds with a gloved finger.

4. Using a scalpel blade cut the vitro-skin segment (3) into two equal sections, each large enough to completely cover the top of the cell. Place the relevant size o-ring (5) (22mm, for the specified Franz-cell) onto each section of the vitro skin and dose to 150mg/$cm^2$ of depilatory composition (4) ("Veet Normal Skin Hair Removal Cream" or an equivalent (an equivalent being a composition comprising 3.7%wt thioglycolic acid)) into the centre then, using a glass rod, evenly spread the cream around the inside of the o-ring (5). Using tweezers pick up the vitro-skin segment and place the vitro-skin segment, depilatory and o-ring centrally over the receptor cell (2), place donor cell (1) over the top and clamp in place. Turn on stirrer plate and start 10 minute countdown timer. After 10 minutes; turn off stirrer and remove the clamp, donor cell (1) and vitro-skin segment and place the receptor solution in a suitable container for analysis.

5. A reference sample should also be run without protective composition treatment on the vitro-skin. Remove a sheet of vitro-skin from the hydration chamber and lay textured side up on a clean flat surface. Repeat step 4 of the protocol to produce the reference sample.

Sample Analysis

For RP-HPLC analysis, prepare a 50mM Formic acid (98%+ Fluka) solution and mix 970ml of this solution with 30ml acetonitrile (HPLC grade) to act as a mobile phase during the analysis.

A reference standard solution should be made with a concentration of Calcium Thioglycolate Trihydrate of 0.94 mg/ml.

Install a Waters Atlantis T3 3μm 4.6 x 50mm column into the HPLC (although any silica- based $C_{18}$ reversed phase RP-HPLC column may be used), and ensure all solvent lines for the RP-HPLC are primed and free of leaks. Allow the mobile phase to circulate through the system for 25 minutes at 0.7mL/Min in order to equilibrate the column. Detection of the thioglycolic acid is via UV spectroscopy.

The RP-HPLC conditions are as follows:

- Injection volume: 20 μL
- Mobile phase flow rate: 0.70 ml/min
- Run time: 10 minutes
- UV Detection wavelength: 240 nm
- Column temperature: 35°C
- UV sampling rate: ≥ 5 per second
- Retention time: Thioglycolic Acid ~ 2.5 min

Calculations:

Calculate the concentration of Thioglycolic Acid in the sample

$$\text{concentration (mg/ml)} = \frac{\textbf{weight of std (mg) x purity}}{\textbf{25}} \times \frac{\textbf{3}}{\textbf{25}}$$

Calculate the concentration of thioglycolic acid in the sample using the following formula:

$$\text{concentration (mg/ml)} = \mathbf{A/B \ x \ C \ x \ E/F}$$

Where,

A = Peak Area of Thioglycolic Acid Sample
B = Average Peak Area of Thioglycolic Acid Standard
C = Thioglycolic Acid final STD concentration in mg/ml (0.94 mg/ml)
E = Molecular weight of Thioglycolic acid (92.12g/mol)
F = Molecular weight of Calcium thioglycolate (184.23g/mol)

The efficacy of the barrier provided by the protective e composition (resistance to TGA penetration) can be calculated as a percentage decrease in TGA in the receptor solution:

$$\%\text{reduction} = \frac{\text{concentration without barrier* – concentration with barrier*}}{\text{concentration without barrier*}} \times 100$$

*protective composition

For example, if TGA in solution without protective composition = 75μg/ml and TGA in solution with barrier = 15 μg/ml

$$\%\text{reduction} = \frac{75 - 60}{75} \times 100 = \mathbf{85\%}$$

A reduction of TGA penetration of 45% or more is believed to correlate to a significant and user-noticeable reduction in irritation.

Examples

| Example | Protective Composition | % Reduction in Thioglycolic Acid Penetration According to the Franz Cell Method |
|---|---|---|
| Inventive Example 1 | 60% Mineral Oil<br>40% Antaron WP660 (Vinyl Pyrolidone/Triacontene Copolymer) | 100.0 |
| Inventive Example 2 | 85% Mineral Oil<br>15% Antaron V220F (Alkylated Polyvinyl Pyrrolidone with Eicosene 30/70 ratio) | 94.0 |
| Inventive Example 3 | 92% Hexamethyldisiloxane<br>6% Acrylate terpolymer (acrylic acid, stearyl methacrylate, isooctyl methacrylate terpolymer)<br>2% Trimethyl phenyl silsesquioxan | 100.0 |
| Comparative Example 1 | 100% mineral oil | 25.0 |
| Comparative Example 2 | 100% Sunflower Seed oil | 10.8 |
| Comparative Example 3 | 100% olive oil | 0.0 |

As demonstrated by the Franz Cell data, oils, on their own (see Comparative Examples 1, 2 and 3) provide little to no barrier to thioglycolic acid, whereas those comprising a copolymer as defined herein present a dramatically superior barrier to penetration (see Inventive Examples 1-3).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

**1.** A depilatory kit comprising:

(a) a protective composition, the protective composition comprising from 1% to 50% copolymer by weight of the protective composition, the copolymer comprising at least 50% by weight of the copolymer of monomers which have hydrophobic side-groups, each hydrophobic side-group comprising from 8 to 40 carbon atoms, wherein the copolymer comprises monomers having formula (monomer A):

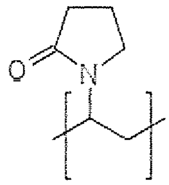

and monomers having formula (monomer B):

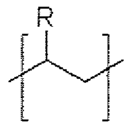

where R is a hydrophobic side-group comprising a straight alkyl chain having from 12 to 30 carbon atoms and wherein the copolymer comprises from 50% to 99%, more preferably 50% to 90% by weight of monomer B, or wherein the copolymer comprises monomers having formula (monomer D):

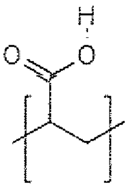

and monomers having formula (monomer E):

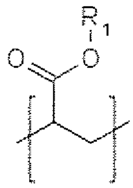

where $R_1$ is a hydrophobic side-group comprising a straight alkyl chain having from 10 to 30 carbon atoms; and monomers having formula (monomer F):

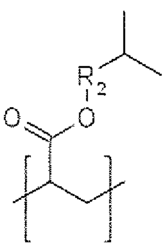

where $R_2$ is a hydrophobic side-group comprising a straight alkyl chain having from 4 to 26 carbon atoms, wherein the copolymer comprises from 50% to 99%, more preferably 50% to 90% by weight of the combination of monomers E and F and
(b) a depilatory composition comprising an effective amount of a keratin reducing agent, comprising a thioglycolate acid salt.

**2.** The kit of claim 1, wherein the protective composition comprises from 1% to 40%, preferably from 2% to 30%, more preferably from 3% to 20% of the copolymer by weight of the protective composition.

**3.** The kit of claim 1 or 2, wherein the copolymer comprises from 50% to 95%, preferably from 60% to 90% by weight

of the copolymer of monomers which have hydrophobic side-groups comprising from 8 to 40 carbon atoms.

**4.** The kit of any preceding claim, wherein each hydrophobic side group comprises from 8 to 30 carbon atoms.

**5.** The kit of any preceding claim, wherein the carbon-carbon bonds within the hydrophobic side-groups have a degree of unsaturation of less than 50%, preferably less than 25% and are more preferably fully saturated.

**6.** The kit of any preceding claim, wherein the hydrophobic side-groups comprise straight chain alkyl groups.

**7.** The kit of any preceding claim, wherein protective composition is an emulsion and the copolymer is comprised within the hydrophobic phase thereof.

**8.** The kit of any preceding claim, wherein the thioglycolic acid salt, comprises potassium or calcium thioglycolate, or mixtures thereof.

**9.** A method of removing hair from skin comprising the steps of:

(a) applying the protective composition defined in any of claims 1 to 7 to an area of skin on which unwanted hair is growing.
(b) applying a depilatory composition to the area of skin to which the protective composition has been applied, the depilatory composition comprising a keratin reducing agent.

**10.** The method of claim 9, comprising the following additional step between step (a) and step (b):

(a1) massaging the protective composition into the skin for at least 10 seconds.

**11.** The method of claim 9 or 10, comprising the following additional step immediately following step (b):

(c) leaving the depilatory composition in place on the protective composition for a period of at least 1 minute, preferably from 1 to 10 minutes, more preferably from 3 to 10 minutes.

**12.** The method of any of claims 9 to 11, wherein the thioglycolic acid salt comprises potassium or calcium thioglycolate, or mixtures thereof.

## Patentansprüche

**1.** Enthaarungskit, das Folgendes umfasst:

(a) eine Schutzzusammensetzung, wobei die Schutzzusammensetzung Copolymer zu 1 Gew.-% bis 50 Gew.-% der Schutzzusammensetzung umfasst, wobei das Copolymer zu mindestens 50 Gew.-% des Copolymers Monomere mit hydrophoben Seitengruppen umfasst, wobei jede hydrophobe Seitengruppe aus 8 bis 40 Kohlenstoffatomen besteht, wobei das Copolymer Monomere der folgenden Formel (Monomer A) aufweist:

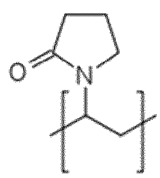

und Monomere, die folgende Formel (Monomer B) aufweisen:

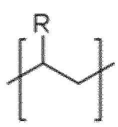

wobei R eine hydrophobe Seitengruppe ist, die eine gerade Alkylkette mit 12 bis 30 Kohlenstoffatomen umfasst, und wobei das Copolymer zu 50 Gew.-% bis 99 Gew.-%, mehr bevorzugt 50 Gew.-% bis 90 Gew.-% Monomer B umfasst, oder wobei das Copolymer Monomere mit der folgenden Formel (Monomer D) umfasst:

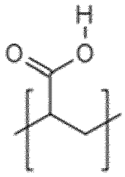

und Monomere, die folgende Formel (Monomer E) aufweisen:

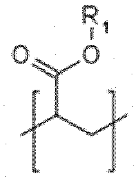

wobei $R_1$ eine hydrophobe Seitengruppe ist, die eine gerade Alkylkette mit 10 bis 30 Kohlenstoffatomen umfasst; und Monomere, die folgende Formel (Monomer F) aufweisen:

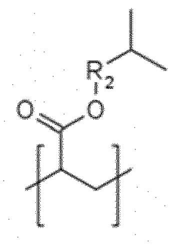

wobei $R_2$ eine hydrophobe Seitengruppe ist, die eine gerade Alkylkette mit 4 bis 26 Kohlenstoffatomen umfasst, wobei das Copolymer zu 50 % bis 99 % Gew.-%, mehr bevorzugt 50 Gew.-% bis 90 Gew.-% die Kombination aus Monomeren E und F umfasst und
(b) eine Enthaarungszusammensetzung, die eine wirksame Menge eines Keratinreduktionsmittels umfasst, das ein Thioglycolatsäuresalz umfasst.

**2.** Kit nach Anspruch 1, wobei die Schutzzusammensetzung Copolymer zu 1 Gew.-% bis 40 Gew.-%, vorzugsweise 2 Gew.-% bis 30 Gew.-%, mehr bevorzugt 3 Gew.-% bis 20 Gew.-% der Schutzzusammensetzung umfasst.

**3.** Kit nach Anspruch 1 oder 2, wobei das Copolymer zu 50 Gew.-% bis 95 Gew.-%, vorzugsweise 60 Gew.-% bis 90 Gew.-% des Copolymers Monomere umfasst, die hydrophobe Seitengruppen aufweisen, die 8 bis 40 Kohlenstoffatome umfassen.

**4.** Kit nach einem der vorstehenden Ansprüche, wobei jede hydrophobe Seitengruppe 8 bis 30 Kohlenstoffatome umfasst.

**5.** Kit nach einem der vorstehenden Ansprüche, wobei die Kohlenstoff-Kohlenstoff-Bindungen innerhalb der hydrophoben Seitengruppen einen Ungesättigtheitsgrad von weniger als 50 %, vorzugsweise weniger als 25 % aufweisen und mehr bevorzugt vollständig gesättigt sind.

**6.** Kit nach einem der vorstehenden Ansprüche, wobei die hydrophoben Seitengruppen geradkettige Alkylgruppen umfassen.

**7.** Kit nach einem der vorstehenden Ansprüche, wobei die Schutzzusammensetzung eine Emulsion ist und das Copolymer innerhalb der hydrophoben Phase davon enthalten ist.

8. Kit nach einem der vorstehenden Ansprüche, wobei das Thioglycolsäuresalz Kalium- oder Kalziumthioglycolat oder Mischungen davon umfasst.

9. Verfahren zum Entfernen von Haaren von der Haut, das die folgenden Schritte umfasst:

(a) Auftragen der Schutzzusammensetzung, die in einem der Ansprüche 1 bis 7 definiert ist, auf einen Hautbereich, auf dem unerwünschte Haare wachsen.
(b) Auftragen der Enthaarungszusammensetzung auf den Hautbereich, auf dem die Schutzzusammensetzung aufgetragen wurde, wobei die Enthaarungszusammensetzung ein Keratinreduktionsmittel umfasst.

10. Verfahren nach Anspruch 9, das den folgenden zusätzlichen Schritt zwischen Schritt (a) und Schritt (b) umfasst:

(a1) Einmassieren der Schutzzusammensetzung in die Haut mindestens 10 Sekunden lang.

11. Verfahren nach Anspruch 9 oder 10, das den folgenden zusätzlichen Schritt unmittelbar nach Schritt (b) umfasst:

(c) Belassen der Enthaarungszusammensetzung auf der Schutzzusammensetzung für eine Dauer von mindestens 1 Minute, vorzugsweise 1 bis 10 Minuten, mehr bevorzugt 3 bis 10 Minuten.

12. Verfahren nach einem der Ansprüche 9 bis 11, wobei das Thioglycolsäuresalz Kalium- oder Kalziumthioglycolat oder Mischungen davon umfasst.

**Revendications**

1. Trousse dépilatoire comprenant :

(a) une composition protectrice, la composition protectrice comprenant de 1 % à 50 % de copolymère en poids de la composition protectrice, le copolymère comprenant au moins 50 % en poids du copolymère de monomères qui ont des groupes latéraux hydrophobes, chaque groupe latéral hydrophobe comprenant de 8 à 40 atomes de carbone, dans laquelle le copolymère comprend des monomères ayant la formule (monomère A) :

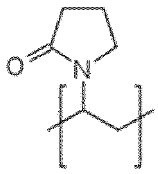

et des monomères ayant la formule (monomère B) :

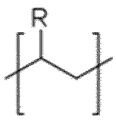

où R est un groupe latéral hydrophobe comprenant une chaîne alkyle linéaire ayant de 12 à 30 atomes de carbone et dans laquelle le copolymère comprend de 50 % à 99 %, plus préférablement 50 % à 90 % en poids de monomère B, ou dans laquelle le copolymère comprend des monomères ayant la formule (monomère D) :

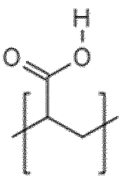

et des monomères ayant la formule (monomère E) :

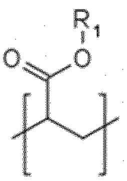

où $R_1$ est un groupe latéral hydrophobe comprenant une chaîne alkyle linéaire ayant de 10 à 30 atomes de carbone ; et des monomères ayant la formule (monomère F) :

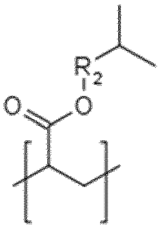

où $R_2$ est un groupe latéral hydrophobe comprenant une chaîne alkyle linéaire ayant de 4 à 26 atomes de carbone, dans laquelle le copolymère comprend de 50 % à 99 %, plus préférablement 50 % à 90 % en poids de la combinaison de monomères E et F et

(b) une composition dépilatoire comprenant une quantité efficace d'un agent réducteur de kératine, comprenant un sel d'acide thioglycolique.

**2.** Trousse selon la revendication 1, dans laquelle la composition protectrice comprend de 1 % à 40 %, de préférence de 2 % à 30 %, plus préférablement de 3 % à 20 % du copolymère en poids de la composition protectrice.

**3.** Trousse selon la revendication 1 ou 2, dans laquelle le copolymère comprend de 50 % à 95 %, de préférence de 60 % à 90 % en poids du copolymère de monomères qui ont des groupes latéraux hydrophobes comprenant de 8 à 40 atomes de carbone.

**4.** Trousse selon une quelconque revendication précédente, dans laquelle chaque groupe latéral hydrophobe comprend de 8 à 30 atomes de carbone.

**5.** Trousse selon une quelconque revendication précédente, dans laquelle les liaisons carbone-carbone au sein des groupes latéraux hydrophobes ont un degré d'insaturation inférieur à 50 %, de préférence, inférieur à 25 % et sont plus préférablement complètement saturées.

**6.** Trousse selon une quelconque revendication précédente, dans laquelle les groupes latéraux hydrophobes comprennent des groupes alkyle à chaîne linéaire.

**7.** Trousse selon une quelconque revendication précédente, dans laquelle la composition protectrice est une émulsion et le copolymère est compris au sein de la phase hydrophobe de celle-ci.

**8.** Trousse selon une quelconque revendication précédente, dans laquelle le sel d'acide thioglycolique, comprend du thioglycolate de potassium ou de calcium, ou des mélanges de ceux-ci.

**9.** Procédé d'élimination de poils de la peau, comprenant les étapes consistant à :

(a) appliquer la composition protectrice définie dans l'une quelconque des revendications 1 à 7 sur une zone de peau sur laquelle poussent des poils indésirables.

(b) appliquer une composition dépilatoire sur la zone de peau sur laquelle la composition protectrice a été appliquée, la composition dépilatoire comprenant un agent réducteur de kératine.

**10.** Procédé selon la revendication 9, comprenant l'étape supplémentaire suivante entre l'étape (a) et l'étape (b) :

(a1) masser la composition protectrice dans la peau pendant au moins 10 secondes.

**11.** Procédé selon la revendication 9 ou 10, comprenant l'étape supplémentaire suivante immédiatement après l'étape (b) :

(c) laisser la composition dépilatoire en place sur la composition protectrice pendant une période d'au moins 1 minute, de préférence de 1 à 10 minutes, plus préférablement de 3 à 10 minutes.

**12.** Procédé selon l'une quelconque des revendications 9 à 11, dans lequel le sel d'acide thioglycolique, comprend du thioglycolate de potassium ou de calcium, ou des mélanges de ceux-ci.

Fig.1.

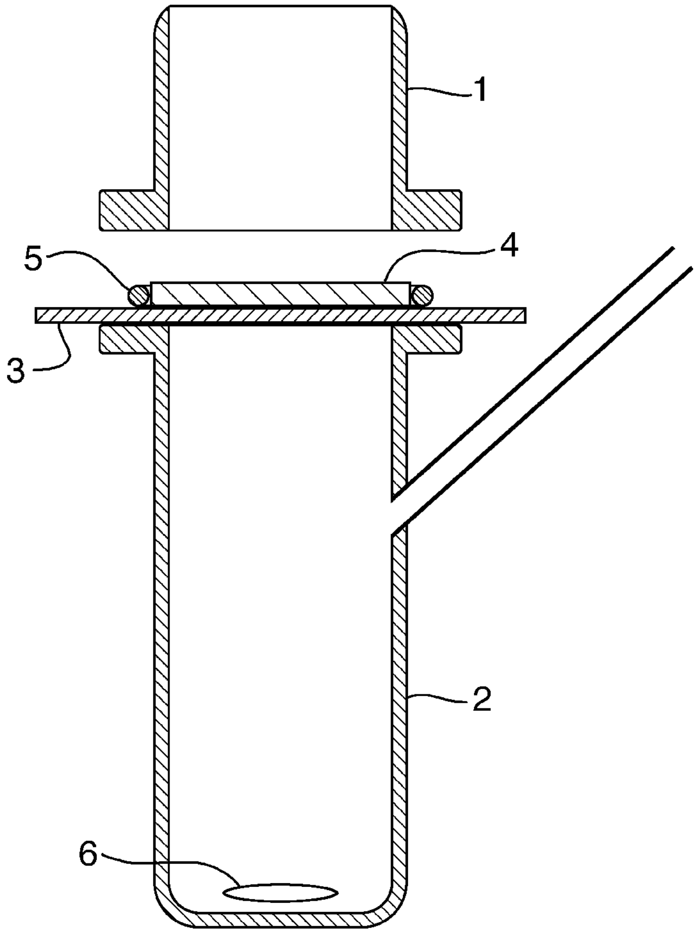

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4401663 A
- US 4424205 A
- US 20040219118 A


### Non-patent literature cited in the description


- McCutcheon's Detergents and Emulsifiers. 1986, 317-324
- The Encyclopaedia of Polymers and Thickeners for Cosmetics. Department of Polymer Science, University of Southern Mississippi